(19) Europäisches Patentamt
European Patent Office
Office européen des brevets

(11) Veröffentlichungsnummer: **0 464 439 A2**

(12) **EUROPÄISCHE PATENTANMELDUNG**

(21) Anmeldenummer: **91109857.2**

(22) Anmeldetag: **15.06.91**

(51) Int. Cl.5: **C07C 29/40**, C07C 31/27, //C07D263/12,C07F7/08, C07C271/12

(30) Priorität: **30.06.90 DE 4020942**

(43) Veröffentlichungstag der Anmeldung: **08.01.92 Patentblatt 92/02**

(84) Benannte Vertragsstaaten: **AT BE CH DE DK ES FR GB GR IT LI LU NL SE**

(71) Anmelder: **BAYER AG**

**W-5090 Leverkusen 1 Bayerwerk(DE)**

(72) Erfinder: **Hoppe, Dieter, Prof. Dr.**
**Schneiderkamp 17e**
**W-2300 Kiel 1(DE)**
Erfinder: **Tebben, Petra**
**Rosenweg 14**
**W-2212 Brunsbüttel(DE)**
Erfinder: **Hintze, Folker**
**Gabelsberger Strasse 2**
**W-3000 Hannover 1(DE)**
Erfinder: **Raffel, Thomas**
**Kopernikusstrasse 83**
**W-2800 Bremen 33(DE)**

(54) **Verfahren zur Herstellung von sec. oder tert. Alkoholen.**

(57) Die Erfindung betrifft ein neues Verfahren zur Herstellung von teilweise bekannten Verbindungen mit sec.- oder tert.-Alkoholgruppen der allgemeinen Formel (I)

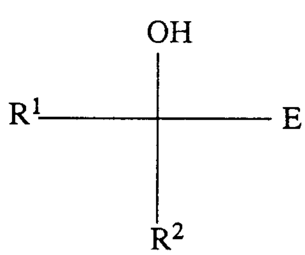

(I)

in welcher $R^1$, $R^2$ und E die in der Beschreibung angegebene Bedeutung haben über $\alpha$-Oxycarbanionen, die wichtige Zwischenprodukte zur Synthese von pharmazeutischen Wirkstoffen, insbesondere zur Synthese von reninhibitorischen und HIV-proteaseinhibitorischen Peptiden sind.

EP 0 464 439 A2

Die Erfindung betrifft ein neues Verfahren zur Herstellung von teilweise bekannten Verbindungen mit sec.- oder tert.-Alkoholgruppen über α-Oxycarbanionen, die wichtige Zwischenprodukte zur Synthese von pharmazeutischen Wirkstoffen, insbesondere zur Synthese von renininhibitorischen und HIV-proteaseinhibitorischen Peptiden sind.

Es ist bereits bekannt, daß α-Oxyalkylcarbanionen als umgepolte Synthone bei der elektrophilen Einführung von α-Hydroxy-alkyl-Resten und bei der Wittigreaktion eingesetzt werden können [vgl. J.E. Saavedra in T.A. Hase (Ed.) "Umpoled Synthons", S. 101, Wiley 1987].

Außerdem ist bekannt, daß die α-Oxyalkylcarbanionen bisher nur durch Spaltung von α-Stannylethern unter Transmetallierung, durch reduktive Spaltung von Monothioacetalen mit Lithiumnaphthalenid oder durch Deprotonierung einiger sterisch gehinderter Benzoate direkt hergestellt werden können [vgl. J. Am. Chem. Soc. 1977, 99, 5213].

Diese Verfahren haben aber aufgrund des Einsatzes von großen Mengen an Basen zur Erhöhung der Deprotonierungsgeschwindigkeit, der schlechten Abspaltbarkeit der Schutzgruppen (Arylcarbonylrest) und der geringen Ausbeute große Nachteile.

Ebenso ist die Darstellung der entsprechenden konfigurationsstabilen, nicht-racemisch chiralen α-Oxyalkyllithiumverbindungen nur über eine stereospezifische Spaltung der enantiomerenreinen Stannane, der meist eine aufwendige Diastereomerentrennung vorgeschaltet ist, möglich.

Die Erfindung betrifft ein Verfahren zur Herstellung von Verbindungen mit sec.- und tert.-Alkoholgruppen der allgemeinen Formel (I)

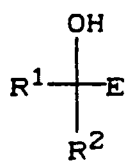

(I)

in welcher

$R^1$

- für Wasserstoff oder für eine elektrofuge Abgangsgruppe steht, oder
- für geradkettiges oder verzweigtes Alkyl oder Alkenyl mit jeweils bis zu 10 Kohlenstoffatomen steht, die gegebenenfalls durch Alkoxy mit bis zu 6 Kohlenstoffatomen, Phenyl oder durch die Gruppe $-NR^3R^4$ substituiert sind,
  worin
  $R^3$ und $R^4$ gleich oder verschieden sind und
- Wasserstoff, geradkettiges oder verzweigtes Alkyl mit bis zu 8 Kohlenstoffatomen, Phenyl oder Benzyl bedeuten,

$R^2$

- für Wasserstoff steht, oder

$R^1$ und $R^2$ gemeinsam einen 3- bis 7-gliedrigen Cycloalkylring bilden

und

E

- für geradkettiges oder verzweigtes Alkyl oder Alkenyl mit jeweils bis zu 8 Kohlenstoffatomen steht, die gegebenenfalls bis zu 3-fach durch Hydroxy, Phenyl oder Cycloalkyl mit 3 bis 7 Kohlenstoffatomen substituiert sind oder durch eine Gruppe der Formel $-NR^3R^4$, $-HN-CO-OR^5$, $-SiR^6R^7R^8$ oder $SnR^{6'}R^{7'}R^{8'}$ substituiert sind,
  worin
  $R^3$ und $R^4$ die oben angegebene Bedeutung haben
  und
  $R^5$
  - geradkettiges oder verzweigtes Alkyl mit bis zu 6 Kohlenstoffatomen bedeutet, das gegebenenfalls durch Phenyl substituiert ist,

  $R^6$, $R^7$, $R^8$, $R^{6'}$, $R^{7'}$ und $R^{8'}$ gleich oder verschieden sind und geradkettiges oder verzweigtes Alkyl mit bis zu 6 Kohlenstoffatomen bedeuten,
- für Cycloalkyl oder Cycloalkenyl mit 3 bis 7 Kohlenstoffatomen steht, das gegebenenfalls durch Hydroxy substituiert ist,
- für Carboxy, Alkoxycarbonyl mit bis zu 4 Kohlenstoffatomen oder für eine Gruppe der Formel $-SiR^6R^7R^8$, $-SnR^{6'}R^{7'}R^{8'}$ oder $R^9-CO$ steht,

worin

$R^6$, $R^{6'}$, $R^7$, $R^{7'}$, $R^8$ und $R^{8'}$ die oben angegebene Bedeutung haben

und

$R^9$

- Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel -NH-CO-OR$^5$ substituiert sind,

  worin

  $R^5$ die oben angegebene Bedeutung hat,

das dadurch gekennzeichnet ist, daß man Carbamate der allgemeinen Formel (II),

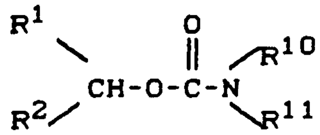

(II)

in welcher

$R^1$ und $R^2$ die oben angegebene Bedeutung haben

und

$R^{10}$ und $R^{11}$ gleich oder verschieden sind und entweder für geradkettiges oder verzweigtes Alkyl mit bis zu 8 Kohlenstoffatomen stehen, oder

gemeinsam mit dem Stickstoffatom für einen Rest der Formel

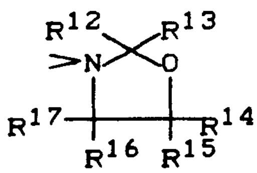

steht,

worin

$R^{12}$, $R^{13}$, $R^{14}$, $R^{15}$, $R^{16}$ und $R^{17}$ gleich oder verschieden sind und für Wasserstoff, geradkettiges oder verzweigtes Alkyl mit bis zu 8 Kohlenstoffatomen, Aryl mit 6 bis 10 Kohlenstoffatomen oder Cycloalkyl mit 3 bis 8 Kohlenstoffatomen stehen, oder jeweils $R^{12}$ und $R^{13}$, $R^{14}$ und $R^{15}$ und/oder $R^{16}$ und $R^{17}$ gemeinsam einen 3 bis 6 gliedrigen Carbocyclus bilden,

in inerten Lösemitteln mit selektiven Basen, in Anwesenheit eines chelatbildenden, gegebenenfalls chiralen Diamins (im folgenden mit X bezeichnet), zu Verbindungen der allgemeinen Formel (III)

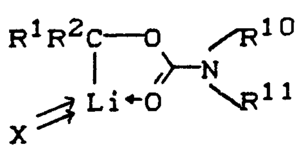

(III)

in welcher

$R^1$, $R^2$, $R^{10}$ und $R^{11}$ die oben angegebene Bedeutung haben,

quantitativ deprotoniert, was im Fall eines chiralen Diamins wie (-)-Spartein enantioselektiv erfolgt,

und dann in inerten Lösemitteln, entweder mit achiralen Elektrophilen der allgemeinen Formeln (IV) und (V) oder mit gegebenenfalls prochiralen Elektrophilen der Formel (VI)

G -Y (IV), $CO_2$ (V), 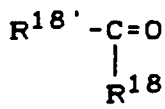 (VI),

in welchen

G - die oben angegebene Bedeutung von E hat und mit diesergleich oder verschieden ist, aber nicht für Wasserstoff oder Carboxy steht,

Y - für Halogen steht,

$R^{18}$ und $R^{18'}$ gleich oder verschieden sind und die oben angegebene Bedeutung von $R^9$ haben oder

$R^{18}$ und $R^{18'}$ gemeinsam einen Cyclobutyl-, Cyclopentyl- oder Cyclohexylring bilden

oder entweder $R^{18}$ oder $R^{18'}$ für eine nukleofuge Abgangsgruppe steht,

substituiert,

und im Fall, daß $R^1$ für eine typische Abgangsgruppe steht, gegebenenfalls unter Ausbildung eines Acylanions und erneuter Deprotonierung nochmals elektrophil substituiert,

und in einem letzten Schritt, gegebenenfalls in Anwesenheit von Hilfsstoffen, die Schutzgruppe -CO-$NR^{10}R^{11}$ solvolytisch abspaltet.

In den vorstehenden Substituentendefinitionen stehen $R^{10}$ und $R^{11}$ vorzugsweise für Alkyl mit bis zu 4 Kohlenstoffatomen, insbesondere für Isopropyl oder gemeinsam mit dem Stickstoffatom für den Rest der Formel

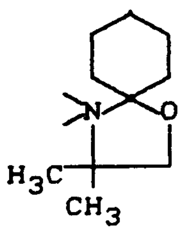

Als bevorzugte chirale Diamine des Substituenten X seien genannt: Tetramethylethylendiamin (TMEDA) und (-)-Spartein.

Halogen steht vorzugsweise für Fluor, Chlor, Brom, insbesondere für Chlor.

Das erfindungsgemäße Verfahren kann durch folgendes Formelschema beispielhaft erläutert werden:

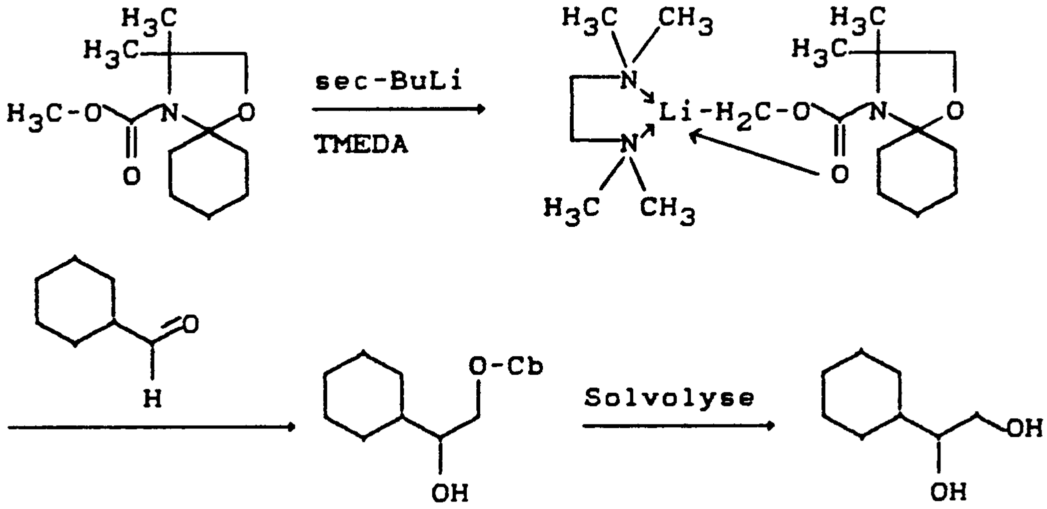


Cb =

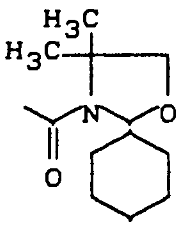

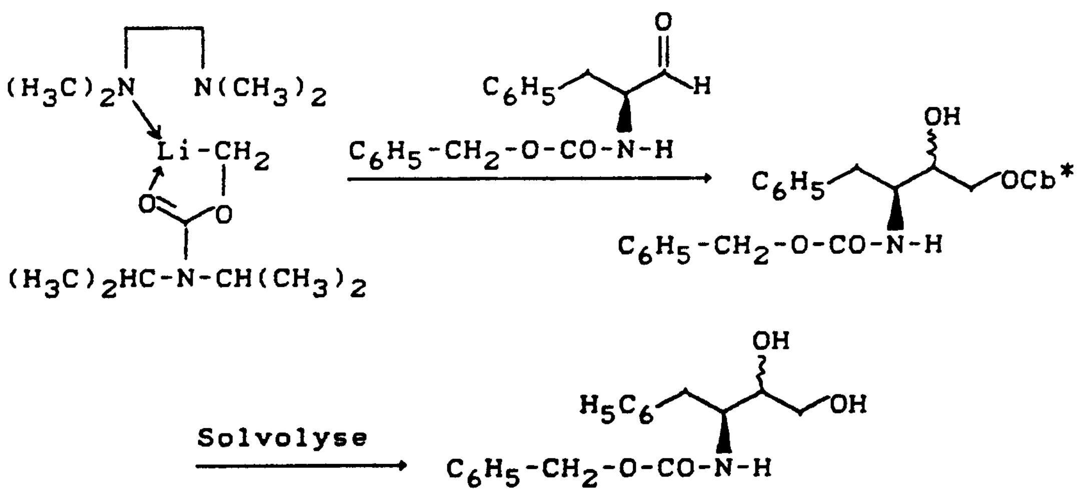

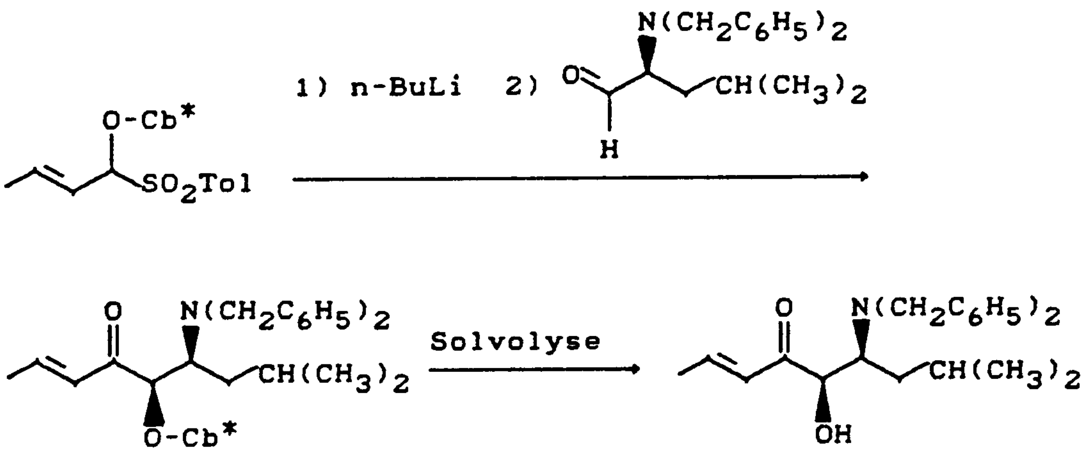


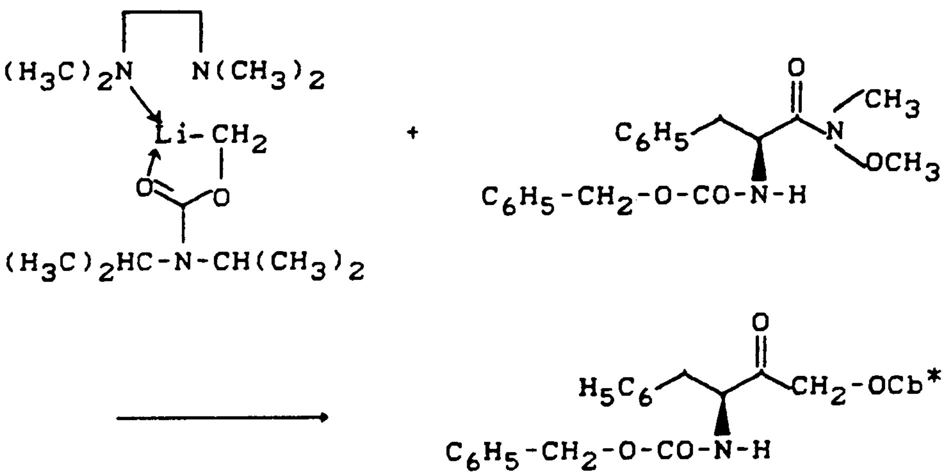

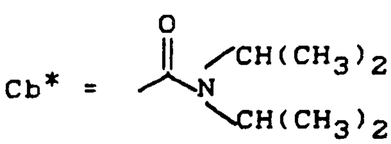


Überraschenderweise liefert das erfindungsgemäße Verfahren die gewünschten Verbindungen der allgemeinen Formel (I) in guten Ausbeuten.

Das Verfahren zeichnet sich durch mehrere Vorteile aus: im Gegensatz zum Stand der Technik können die Carbamidsäureester als Schutzgruppen leicht eingeführt und abgespaltet werden. Außerdem sind stöchiometrische Mengen an Basen, vorzugsweise sec.-Butyllithium für die Deprotonierung ausreichend. Außerdem ermöglicht das erfindungsgemäße Verfahren sowohl eine Steuerung der Diastereoselektivität durch die Wahl von achiralen oder prochiralen elektrophilen Substituenten als auch die Beeinflussung der asymmetrischen Induktion bei prochiralen Carbamaten in Gegenwart von chiralen komplexbildenden Diaminen.

Durch Einsatz von enantiomerenreinen Diaminverbindungen wie beispielsweise (-)-Spartein, erfolgt die Deprotonierung der Verbindungen der Formel (II) enantioselektiv und in sehr guten Ausbeuten zu den entsprechenden chiralen, bevorzugt zu den (S)-Lithiumverbindungen der Formel (III), die durch weitere Umsetzung, beispielsweise mit Chlortrimethylstannan oder Kohlendioxid zu den entsprechenden Carbonsäurederivaten, bevorzugt in der R-Konfiguration überführt werden können.

Die enantioselektive Deprotonierung soll durch folgendes Formelschema beispielhaft erläutert werden:

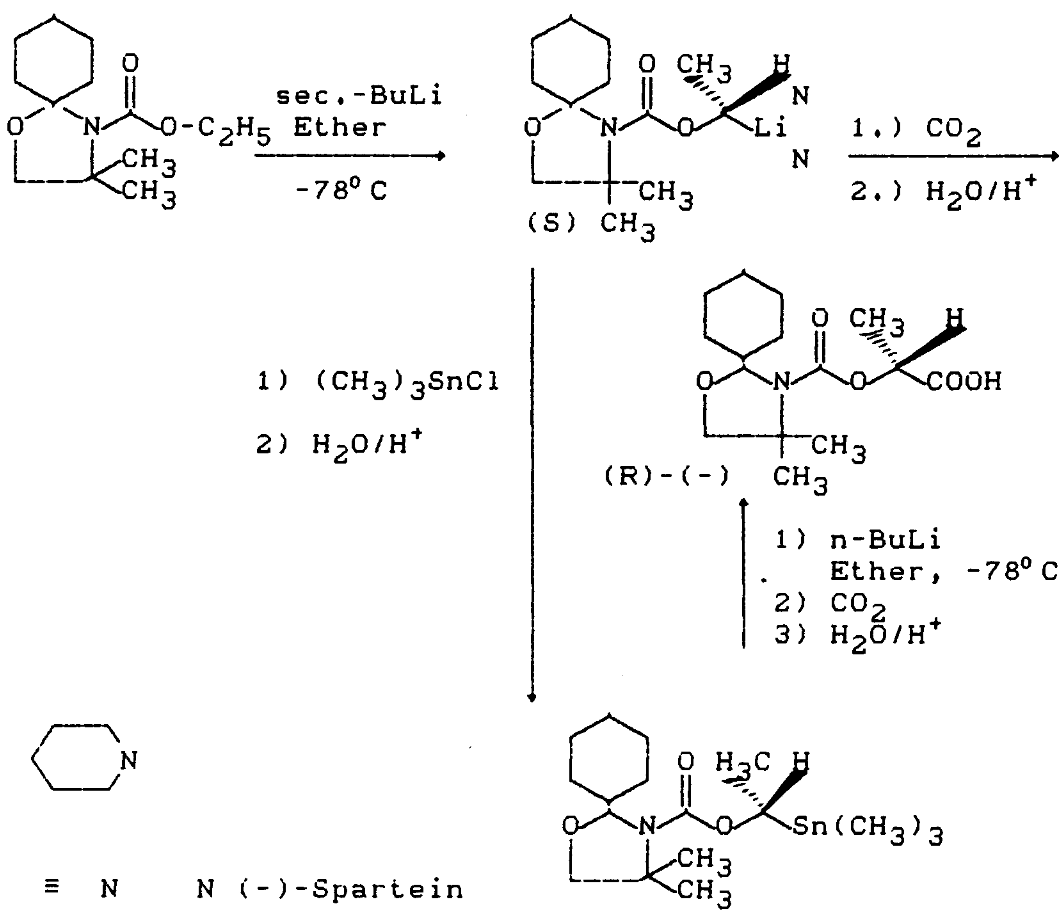


(S)-(+)-5

Die nach dem erfindungsgemäßen Verfahren hergestellten Verbindungen sind durch die Formel (I) allgemein definiert,

Wenn $R^1$ im Rahmen der oben angegebenen Definition für eine typische Abgangsgruppe steht, dann bevorzugt für eine bei Substitutionsreaktionen übliche Gruppe aus der Reihe: Chlor, Brom, Jod, Tosylat, Mesylat oder dem Rest $-OSO_2CF_3$.

Nach dem erfindungsgemäßen Verfahren werden bevorzugt Verbindungen der allgemeinen Formel (I), in welcher

$R^1$

- für Wasserstoff oder für die Gruppe

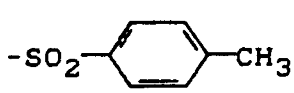

steht,

- für geradkettiges oder verzweigtes Alkyl oder Alkenyl mit jeweils bis zu 8 Kohlenstoffatomen steht, die gegebenenfalls durch Alkoxy mit bis zu 4 Kohlenstoffatomen, Phenyl oder durch die Gruppe $-NR^3R^4$ substituiert sind,

worin

$R^3$ und $R^4$ gleich oder verschieden sind und

- Wasserstoff, geradkettiges oder verzweigtes Alkyl mit bis zu 6 Kohlenstoffatomen oder Benzyl bedeuten,

$R^2$

- für Wasserstoff steht, oder

$R^1$ und $R^2$ gemeinsam den Cyclopentylring bilden

und

E

- für geradkettiges oder verzweigtes Alkyl oder Alkenyl mit jeweils bis zu 6 Kohlenstoffatomen steht, die gegebenenfalls gleich oder verschieden bis zu 3-fach durch Hydroxy, Phenyl, Cyclobutyl, Cyclopentyl, Cyclohexyl oder durch eine Gruppe der Formel $-NR^3R^4$, -NH-CO-$OR^5$, $-SiR^6R^7R^8$ oder $-SnR^{6'}R^{7'}R^{8'}$ substituiert sind,
  worin
  $R^3$ und $R^4$ die oben angegebene Bedeutung haben,
  $R^5$
  - geradkettiges oder verzweigtes Alkyl mit bis zu 4 Kohlenstoffatomen bedeutet, das gegebenenfalls durch Phenyl substituiert ist,

  $R^6$, $R^{6'}$, $R^7$, $R^{7'}$, $R^8$ und $R^{8'}$ gleich oder verschieden sind und geradkettiges oder verzweigtes Alkyl mit bis zu 4 Kohlenstoffatomen bedeuten,
- für Cyclobutyl oder Cyclohexyl steht, die gegebenenfalls durch Hydroxy substituiert sind,
- für Carboxy, Methoxycarbonyl oder für eine Gruppe der Formel $-SiR^6R^7R^8$, $-SnR^{6'}R^{7'}R^{8'}$ oder $R^9$-CO- steht,
  worin
  $R^6$, $R^{6'}$, $R^7$, $R^{7'}$, $R^8$ und $R^{8'}$ die oben angegebene Bedeutung haben
  und
  $R^9$
  - Wasserstoff oder geradkettiges oder verzweigtes Alkyl oder Alkenyl mit jeweils bis zu 6 Kohlenstoffatomen bedeutet, die gegebenenfalls bis zu 3-fach durch Phenyl oder durch die Gruppe der Formel -NH-CO-$OR^5$ substituiert sind,
    worin
    $R^5$ die oben angegebene Bedeutung hat,

hergestellt.

Besonders bevorzugt werden nach dem erfindungsgemäßen Verfahren Verbindungen der allgemeinen Formel (I) hergestellt,
in welcher

$R^1$

- für Wasserstoff oder Alkyl mit 1-4 C-Atomen steht,

$R^2$

- für Wasserstoff steht,

E

- für geradkettiges oder verzweigtes Alkyl oder Alkenyl mit jeweils bis zu 4 Kohlenstoffatomen steht, die gegebenenfalls gleich oder verschieden bis zu 3-fach durch Hydroxy, Phenyl, Cyclobutyl, Cyclohexyl oder durch eine Gruppe der Formel - $NR^3R^4$, -NHCO-$OR^5$, $-SiR^6R^7R^8$ oder $-SnR^{6'}R^{7'}R^{8'}$ substituiert sind
  worin
  $R^3$ und $R^4$ gleich oder verschieden sind und Wasserstoff, geradkettiges oder verzweigtes Alkyl mit bis zu 4 Kohlenstoffatomen oder Benzyl bedeuten,
  $R^5$
  - Methyl oder Ethyl bedeutet, die gegebenenfalls durch Phenyl substituiert sind,

  $R^6$, $R^{6'}$, $R^7$, $R^{7'}$, $R^8$ und $R^{8'}$ für Methyl stehen,
- für Cyclobutyl oder Cyclohexyl steht, die gegebenenfalls durch Hydroxy substituiert sind,
- für Carboxy, Methoxycarbonyl oder für eine Gruppe der Formel $-SiR^6R^7R^8$, $-SnR^{6'}R^{7'}R^{8'}$ oder $R^9$-CO- steht,
  worin
  $R^6$, $R^{6'}$, $R^7$, $R^{7'}$, $R^8$ und $R^{8'}$ die oben angegebene Bedeutung haben
  und
  $R^9$
  - Wassserstoff oder geradkettiges oder verzweigtes Alkyl oder Alkenyl mit jeweils bis zu 4 Kohlenstoffatomen bedeutet, die gegebenenfalls bis zu 3-fach durch Phenyl oder durch die Gruppe der Formel -NH-CO-$OR^5$ substituiert sind,
    worin
    $R^5$ die oben angegebene Bedeutung hat.

Als Lösemittel für die Deprotonierung eignen sich bevorzugt inerte organische Lösemittel wie Kohlenwasserstoffe wie Hexan, Pentan, Ligroin oder Toluol, und Ether, beispielsweise Tetrahydrofuran, Diethylether, Dioxan, Dimethoxyethan, Diglyme, Triglyme oder tert.-Butylmethylether. Besonders bevorzugt sind Tetrahydrofuran und Diethylether.

Die Deprotonierung erfolgt in einem Temperaturbereich von -100 °C bis Raumtemperatur, vorzugsweise bei ca. -78 °C bis 0 °C.

Die Deprotonierung kann sowohl bei Normaldruck als auch bei erhöhtem oder erniedrigtem Druck (z.b. 0,5 bis 2 bar), vorzugsweise bei Normaldruck durchgeführt werden.

Als selektive Basen eignen sich Alkyllithiumverbindungen mit bis zu 6 C-Atomen in der Alkylgruppe, vorzugsweise n-Butyllithium oder sec.-Butyllithium.

Die Base wird in einer Menge von 0,5 bis 5 mol, vorzugsweise in stöchiometrischen Mengen eingesetzt.

Die elektrophile Substitution erfolgt ebenfalls in den oben aufgeführten Lösemitteln, bevorzugt in Tetrahydrofuran bei Normaldruck.

Die elektrophile Substitution wird in einem Temperaturbereich von ca. -100 °C bis +40 °C, vorzugsweise im Bereich von -78 °C bis Raumtemperatur durchgeführt.

Die Abspaltung der Schutzgruppen (Carbamidsäureester) erfolgt nach üblicher Methode durch sequentielle Behandlung mit Säuren und Basen bevorzugt in Methanol.

Als Säuren eignen sich starke anorganische Säuren und organische Sulfon- oder Carbonsäuren wie beispielsweise Methansulfonsäure, Ethansulfonsäure, Benzolsulfonsäure, Toluolsulfonsäure, Essigsäure oder Propionsäure.

Als Basen eignen sich sich Alkali- und Erdalkalihydroxide wie beispielsweise Natrium-, Kalium- oder Bariumhydroxid. Bevorzugt ist Bariumhydroxid.

Die Säuren und Basen werden in einer Menge von 0,01 bis 10 mol, vorzugsweise 1 mol eingesetzt.

Die Abspaltung der Carbamatschutzgruppen erfolgt bei Normaldruck in einem Temperaturbereich von 0 °C bis +130 °C, vorzugsweise von +20 °C bis +100 °C.

Die enantioselektive Deprotonierung verläuft unter den Bedingungen (Lösemittel, Temperatur, Druck), die für die Deprotonierung oben aufgeführt wurden.

Die Verbindungen der allgemeinen Formel (II) sind an sich bekannt oder können beispielsweise hergestellt werden, indem man Amine der allgemeinen Formel

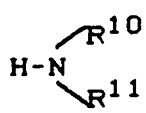

mit Verbindungen der allgemeinen Formel

$Z\text{-}CO\text{-}O\text{-}CHR^1R^2$

in welchen

$R^1$, $R^2$, $R^{10}$ und $R^{11}$ die oben angegebene Bedeutung haben

und

Z - für Chlor steht,

in inerten Lösemitteln umsetzt.

Beispielhaft sei die Herstellung der Ausgangsverbindungen der Formel (II) im folgenden beschrieben:
Zu einer Lösung von 4,73 g (50,0 mmol) Chlorameisensäureethylester in 50 ml Methylenchlorid werden bei Raumtemperatur 16,9 g 3,3-Dimethyl-1-oxa-4-azaspiro[4,5]decan zugegeben. Nach 15-stündigem Nachrühren wird die Reaktionsmischung auf 2 N Salzsäure gegeben, die wäßrige Phase zweimal ausgeethert und dann mit gesättigter Natriumhydrogencarbonatlösung neutralisiert. Man erhält die Verbindung Methyl-3,3-dimethyl-1-oxa-4-azaspiro[4,5]decan-4-carboxylat der Formel

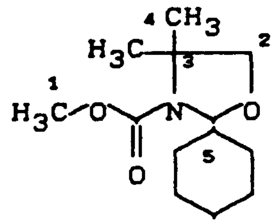

Sdp. 125° C / 8 Torr.
Schmp.: 32-33° C (a.d. Schmelze)
$R_f$ = 0,63 (Ether/n-Pentan = 1:1)
IR (KBr): 1700 $cm^{-1}$ (C=O)
In analoger Weise wird die Verbindung Ethyl-3,3-dimethyl-1-oxa-4-azaspiro[4,5]decan-4-carboxylat

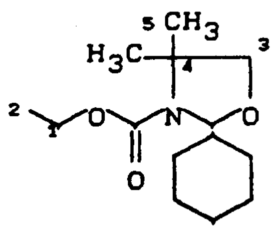

erhalten (Ausbeute: 77 % d. Th.).
Sdp. 165° C/8 Torr.
Schmp.: 42° C (a.d. Schmelze)
$R_f$ = 0,62 (Ether/n-Pentan = 1:1)
IR (KBr): 1690 $cm^{-1}$ (C=O)
Durch Umsetzung von 3,3-Dimethyl-1-oxa-4-azaspiro[4,5]decan mit Trichlormethylchloroformiat erhält man das entsprechende Carbonylchlorid der Formel

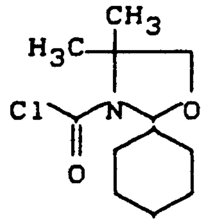

welches zu einer Suspension von Natriumhydrid (80 %iges Parafinöl) in wasserfreiem Ether und Isopropanol injiziert wird und ca. 4 Stunden bei Raumtemperatur gerührt wird. Man erhält 1-Methylethyl-3,3-dimethyl-1-oxa-4-azaspiro[4,5]decan-4-carboxylat der Formel

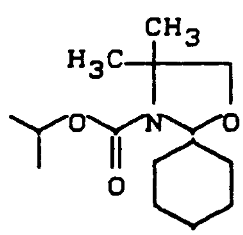

Ausbeute: 90 % d. Th.

In analoger Weise wurde N-(benzyloxycarbonyl)-(S)-phenylalaninal der Formel

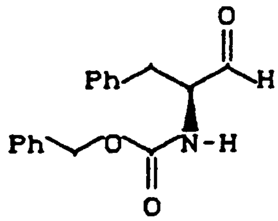

erhalten.

Allgemeine Arbeitsvorschrift I zur Metallierung der Carbamate und Umsetzung des Anions mit Chlortrimethylsilan

1,0 Äquivalente Carbamat und 1,1 Äquivalente TMEDA werden in wasserfreiem Ether (3 ml/mmol) gelöst und bei -78°C mit 1,1 Äquivalenten einer ca. 1,2N Lösung Base (z.B. sec.-BuLi) in Cyclohexan/Isopentan versetzt. Zur vollständigen Lithiierung wurde die Reaktionsmischung 1 - 5 h nachgerührt, dann 1,1 Äquivalente Chlortrimethylsilan injiziert und eine weitere Stunde bei -78°C nachgerührt. Zur Aufarbeitung gibt man die Reaktionsmischung auf 2N Salzsäure und Ether (je 10 ml/mmol), trennt die Phasen, extrahiert die wäßrige Phase noch zweimal mit Ether, entsäuert mit ges. Natriumhydrogencabonat-Lösung, trocknet über Magnesiumsulfat und zieht das Solvens im Vakuum ab. Die Reinigung des Rohprodukts erfolgt flüssigkeitschromatographisch an Kieselgel mit Ether/Pentan-Gemischen.

Allgemeine Arbeitsvorschrift II

Metallierung mit n-BuLi

227 mg (1,0 mmol) Methyl-3,3-dimethyl-1-oxa-4-azaspiro[4,5]decan-4-carboxylat werden mit 1,1 ml n-BuLi (ca. 1,6N in n-Hexan) und nach 5 h mit 138 mg (1,1 mmol) Chlortrimethylsilan versetzt. Nach Aufarbeitung analog der allgemeinen Arbeitsvorschrift I erhält man 60 mg (20% der Theorie) der Verbindung Trimethylsilylmethyl-3,3-dimethyl-1-oxa-4-azaspiro[4,5]decan-4-carboxylat der Formel

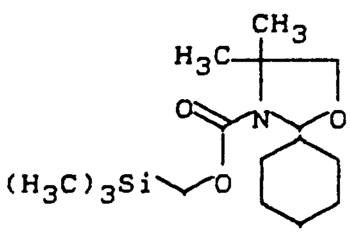

neben 160 mg (70% der Theorie) der Verbindung Methyl-3,3-dimethyl-1-oxa-4-azaspiro[4,5]decan-4-carboxylat der Formel

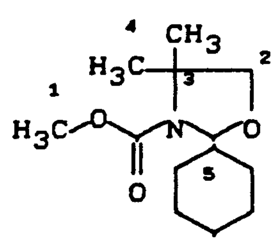

Analog dieser Arbeitsvorschrift II wurde die Verbindung 1-Trimethylsilylethyl-3,3-dimethyl-1-oxa-4-azaspiro-[4,5]decan-4-carboxylat der Formel

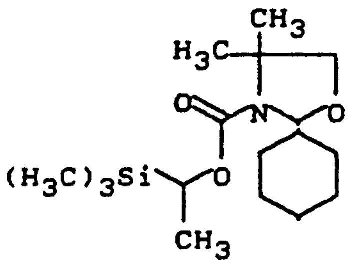

erhalten.

Allgemeine Arbeitsvorschrift III

1,0 Äquivalente Carbamat und 1,1 Äquivalente TMEDA werden in wasserfreiem Ether (3 ml/mmol) gelöst und bei -78° C mit 1,1 Äquivalente einer ca. 1,2N Lösung von sec.-BuLi in Cyclohexan/Isopentan versetzt. Zur vollständigen Lithiierung wird die Reaktionsmischung 1 - 5 h nachgerührt, dann 1,1 Äquivalente des Elektrophils injiziert und 3 - 15 h bei -78° C nachgerührt. Zur Aufarbeitung gibt man die Reaktionsmischung auf 2N Salzsäure und Ether (je 10 ml/mmol), trennt die Phasen, extrahiert die wäßrige Phase noch zweimal mit Ether, entsäuert mit gesättigter Natriumhydrogencarbonat-Lösung, trocknet über Magnesiumsulfat und zieht das Solvens im Vakuum ab. Die Reinigung des Rohprodukts erfolgt flüssigkeitschromatographisch an Kieselgel mit Ether/Pentan-Gemischen.

Nach dem Verfahren der Arbeitsvorschrift III wurden folgende Verbindungen hergestellt:
(2-Hydroxy-3-methylen-4-methylpentyl)-3,3-dimethyl-1-oxa-4-azaspiro[4.5]decan-4-carboxylat

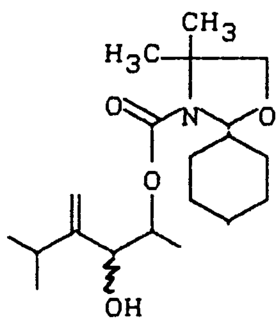

(2-Hydroxy-2-cyclohexylethyl)-3,3-dimethyl-1-oxa-4-azaspiro[4,5]decan-4-carboxylat

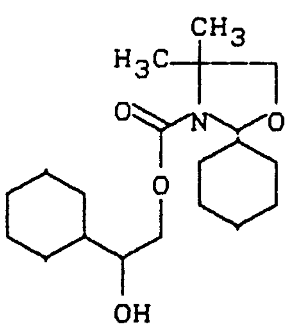

3-Butenyl-3,3-dimethyl-1-oxa-4-azaspiro[4,5]decan-4-carboxylat

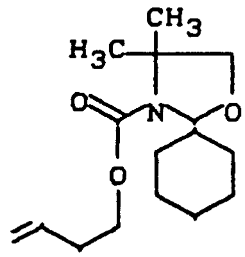

(2-Hydroxy-1,2-dimethylpropyl)-3,3-dimethyl-1-oxa-4-azaspiro[4,5]decan-4-carboxylat

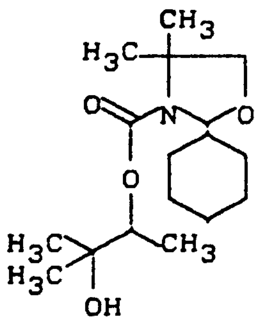

(2-Hydroxy-3-methylen-1,4-dimethylpentyl)-3,3-dimethyl-1-oxa-4-azaspiro[4,5]decan-4-carboxylat

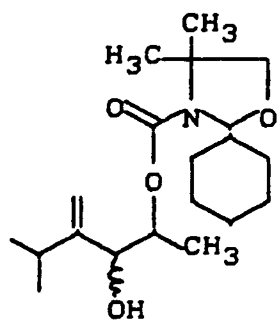

(3S)-[3-(N-Benzyloxycarbonylamino)-2-oxo-4-phenylbutyl]-N,N-diisopropylcarbamidat

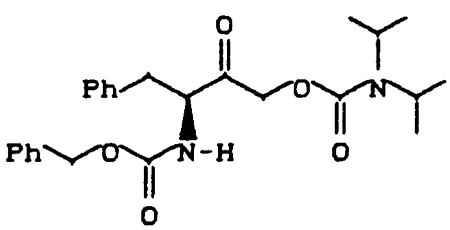

[(3S)-3-(N-Benzyloxycarbonylamino)-2-oxo-4-phenylbutyl]-3,3-dimethyl-1-oxa-4-azaspiro[4.5]decan-4-carboxylat

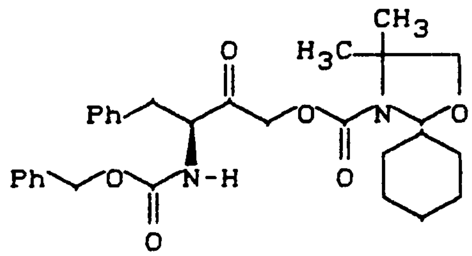

(R)-(-)-2-[(3,3-Dimethyl-1-oxa-4-azaspiro[4.5]dec-4-yl-carbonyl)oxy]propansäure

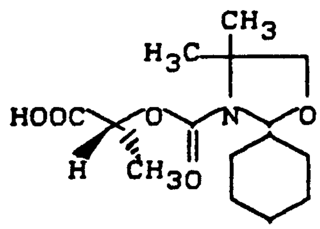

(S)-( + )-2-(Trimethylstannyl)ethyl-3,3-dimethyl-1-oxa-2-azaspiro[4.5]decan-carboxylat

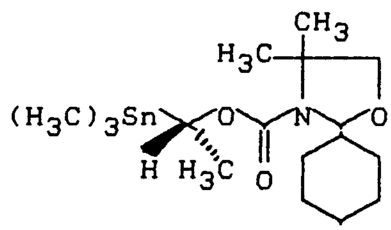

(R)-(-)-Methyl-2-[(3,3-dimethyl-1-oxa-4-azaspiro[4.5]dec-4-yl-carbonyl)oxy]propanoat

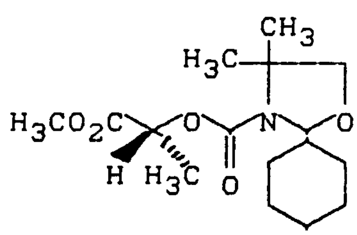

Allgemeine Arbeitsvorschrift IV

Zu einer auf -78° C gekühlten Lösung von 2.8 mmol sec-BuLi (in Cyclohexan/Isopentan) in 8 ml Ether werden 2.9 mmol (-)-Spartein gegeben. Nach 10 min. Rühren injiziert man eine Lösung von 2.0 mmol Ethyl-3,3-dimethyl-1-oxa-4-azaspiro[4,5]decan-4-carboxylat in 2 ml Ether und rührt 5 - 6 Stunden bei -78° C. Nach dem Einleiten von überschüssigem Kohlendioxid in die Lösung der obengenannten Verbindung als (-)-Sparteinkomplex (für die Synthese der Beispiele 4 - 10) oder Zugabe von 3.5 mmol Trimethylzinnchlorid bzw. Methyliodid (für die Synthese der Beispiele 11 - 15) wird das Reaktionsgemisch 16 Stunden bei -78° C gerührt. Zur Aufarbeitung gießt man es nach dem Erwärmen auf Raumtemperatur auf 10 ml Ether/10 ml 2N HCl, extrahiert zweimal mit je 20 ml Ether, trocknet die vereinigten organischen Phasen über $NaHCO_3/Na_2SO_4$ (1:2) bzw. nur über $Na_2SO_4$ (für die Beispiele 4 - 10) und reinigt das Rohprodukt durch Flash-Chromatographie an Kieselgel mit Ether/Pentan-Gemischen. Die Racemate rac-TMEDA generiert man auf die gleiche Weise, jedoch mit 2.9 mmol TMEDA anstelle von (-)-Spartein.

Zur Abspaltung der Cbx-Gruppe werden 2 mmol der Verbindungen aus den Beispielen 4 - 15 mit 10 ml

Methanol und 0.1 ml Methansulfonsäure versetzt und 16 Stunden unter Rückfluß gehalten. Anschließend gibt man 1.0 g $Ba(OH)_2.8\ H_2O$ hinzu und kocht die Mischung weitere 4 Stunden unter Rückfluß. Zur Aufarbeitung gießt man sie auf 10 ml 2N HCl (die gegebenenfalls mit KCl gesättigt wird) extrahiert dreimal mit je 20 ml Ether, trocknet die Etherlösung über $Na_2SO_4$ und reinigt das Rohprodukt an Kieselgel durch Flash-Chromatographie.

Die in den Tabellen 1 - 4 aufgeführten Beispiele werden nach der Arbeitsvorschrift IV hergestellt.

Tabelle 1

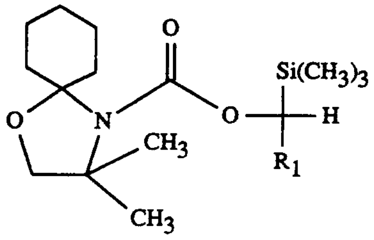

| Bsp. Nr. | $R_1$ | Elektrophil | Ausbeute (% d.Th.) | $[\alpha]_D^{20-23}$ | ee[%] |
|---|---|---|---|---|---|
| (rac) 1[a] | H | $(CH_3)_3SiCl$ | 97 | - | - |
| (rac) 2[a] | $CH_3$ | $(CH_3)_3SiCl$ | 70 | - | - |
| (S) 3 | $CH_3$ | $(CH_3)_3SiCl$ | 67 | -22,5[b] | - |

Tabelle 2

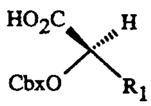

| Bsp. Nr. | $R_1$ | Elektrophil | Ausbeute (% d.Th.) | $[\alpha]_D^{20-23}$ | ee[%] |
|---|---|---|---|---|---|
| (rac) 4[a] | $CH_3$ | $CO_2$ | 60 | - | – |
| (R) 5 | $CH_3$ | $CO_2$ | 75 | - 22,3[c] | > 95 |
| (rac) 6[a] | $(CH_2)_2CH_3$ | $CO_2$ | 65 | - | - |
| (R) 7 | $(CH_2)_2CH_3$ | $CO_2$ | 78 | - 12,6[c] | > 95 |
| (R) 8 | $(CH_2)_5CH_3$ | $CO_2$ | 62 | - | - |
| (R) 9 | $(CH_2)_5CH_3$ | $CO_2$ | 70 | - 12,4[c] | > 95 |
| (R) 10 | $CH(CH_3)_2$ | $CO_2$ | 52 | - 17,3[c] | > 95 |

Tabelle 3

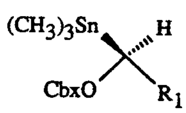

| Bsp. Nr. | $R_1$ | Elektrophil | Ausbeute (% d.Th.) | $[\alpha]_D^{20-23}$ | ee[%] |
|---|---|---|---|---|---|
| (S) 11 | $CH_3$ | $(CH_3)_3SnCl$ | 76 | + 35,4[b] | > 95 |
| (S) 12 | $(CH_2)_2CH_3$ | $(CH_3)_3SnCl$ | 62 | + 35,2[b] | > 95 |
| (S) 13 | $(CH_2)_5CH_3$ | $(CH_3)_3SnCl$ | 86 | + 34,2[b] | > 95 |
| (S) 14 | $CH(CH_3)_2$ | $(CH_3)_3SnCl$ | 62 | + 23,5[b] | > 95 |

Tabelle 4

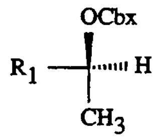

| Bsp. Nr. | $R_1$ | Elektrophil | Ausbeute (% d.Th.) | $[\alpha]_D^{20-23}$ | ee[%] |
|---|---|---|---|---|---|
| (S) 15 | $(CH_2)_5CH_3$ | $CH_3J$ | 81 | + 14,1[c] | 96 |

CbxO= 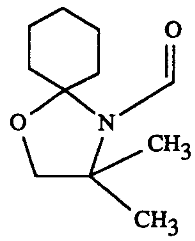

Cl = mit TMEDA als Ligand

b = c = 1,4 - 2,8, $CH_2Cl_2$ ($R_f$)

c = c = 1,1 - 1,9, Aceton ($R_f$)

Die folgenden Ausführungsbeispiele stehen beispielhaft für die Herstellung der Verbindungen der allgemeinen Formel (I) gemäß dem erfindungsgemäßen Verfahren:

Ausführungsbeispiele

Beispiel I

(1-Hydroxycyclohexyl)methyl-N,N-diisopropylcarbamidat

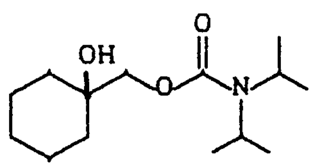

Zu 318 mg (2 mmol) Methyl-N,N-diisopropylcarbamidat und 355 mg TMEDA in 6 ml THF gibt man 2,2 mmol sec.-BuLi bei einer Innentemperatur von -70 °C bis -78 °C. Nach 45 min. versetzt man die Reaktionsmischung mit 98 mg (1 mmol) Cylohexanon (alle 15 min ca. 25 mg). Nach 4 h ist die Reaktion beendet und der Ansatz wird in der Kälte mit 10 ml 2N Salzsäure und 15 ml Ether versetzt, auf Raumtemperatur erwärmt und die Phasen getrennt. Die wässrige Phase wird zweimal ausgeethert und die vereinigten organischen Phasen mit gesättigter $NaHCO_3$-Lösung entsäuert. Nach der Trocknung über Magnesiumsulfat wird das Lösemittel im Vakuum abgezogen und man erhält nach der chromatographischen Reinigung an Kieselgel mit Ether /Petrolether (1:2) 190 mg (74% der Theorie) der Titelverbindung als farblose Kristalle.
$R_f$ = 0,26 (Ether/Petrolether = 1:1, Kieselgel)
Schmp.: 79 °C (n-Pentan)

Beispiel II

(1-Hydroxycyclobutyl)methyl-N,N-diisopropylcarbamidat

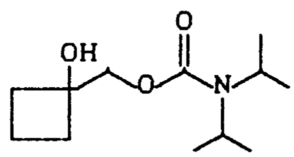

11,148 g (70 mmol) Methyl-N,N-diisopropylcarbamidat und 12,424 g (105 mmol) TMEDA in 200 ml THF werden bei -78° C mit 70 mmol sec, BuLi lithiiert. Nach 1 h gibt man 2,453 g (35 mmol) Cyclobutanon portionsweise (alle 30 min ca. 500 mg) hinzu, Nach 3 h ist die Reaktion beendet (DC-Kontrolle) und die Reaktionsmischung wird in der Kälte mit 100 ml Ether und 120 ml 2N Salzsäure versetzt. Nach dem Erwärmen auf Raumtemperatur trennt man die Phasen und ethert die wässrige Phase dreimal mit je 50 ml Ether aus, Die vereinigten organischen Phasen werden anschließend mit 50 ml gesättigter $NaHCO_3$-Lösung entsäuert und über Magnesiumsulfat getrocknet. Nachdem das Solvens im Vakuum abgezogen ist, erhält man eine Rohausbeute von 12,197 g, dessen chromatographische Reinigung an Kieselgel mit Ether/Petrolether (1:1) 3,721 g (46,4% der Theorie) der Titelverbindung als farblose Flüssigkeit ergibt.
$R_f$ = 0,24 (Ether/Petrolether = 1:1, Kieselgel)
IR (Film); 3430 (OH) und 1670 $cm^{-1}$ (OCON)

| $C_{12}H_{23}NO_3$ (229,32) | | |
|---|---|---|
| Ber.: | C 62,85 | H 10,11 |
| Gef.: | C 62,86 | H 10,24 |

Beispiel III

rac-(2-Hydroxy-3-methylbutyl)-N,N-diisopropylcarbamidat

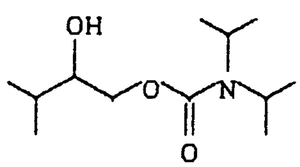

478 mg (3 mmol) Methyl-N,N-diisopropylcarbamidat werden mit 392 mg (3,3 mmol) TMEDA in 4 ml THF bei -78° C mit 3,3 mmol sec-BuLi lithiiert. Nach 1 h versetzt man die Reaktionsmischung mit 144 mg (2 mmol) Isobutyraldehyd und rührt 21 h lang. Danach wird die Reaktionsmischung bei -78° C mit 10 ml 2N Salzsäure und 10 ml Ether versetzt und auf Raumtemperatur erwärmt. Man trennt die Phasen, ethert die wäßrige Phase zweimal aus und neutralisiert die vereinigten organischen Phasen mit gesättigter $NaHCO_3$-Lösung. Nach dem Trocknen über Magnesiumsulfat zieht man das Lösemittel ab und reinigt das Rohprodukt von 500 mg chromatographisch an Kieselgel mit Ether/Petrolether (1:3). Man erhält 299 mg (64,6% der Theorie) der Titelverbindung als farblose Flüssigkeit.
$R_f$ = 0,26 (Ether/Petrolether = 1:1, Kieselgel)
IR (Film): 3400 (OH) und 1670 $cm^{-1}$ (OCON)

| $C_{12}H_{25}NO_3$ (231,34) | | |
|---|---|---|
| Ber.: | C 62,30 | H 10,89 |
| Gef.: | C 62,39 | H 10,89 |

Beispiel IV und Beispiel V

(2S,3S)-3-(N-Benzyloxycarbonylamino)-2-hydroxy-4-phenylbutyl-N,N-diisopropylcarbamidat (Beispiel IV) und (2R,3S)-3-(N-Benzyloxycarbonylamino)-2-hydroxy-4-phenylbutyl-N,N-diisopropylcarbamidat (Beispiel V)

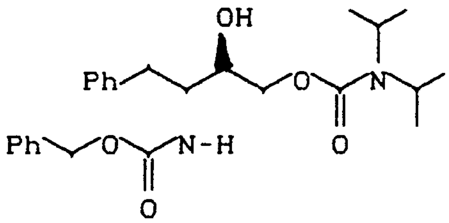

478 mg (3 mmol) Methyl-N,N-diisopropylcarbamidat und 392 mg (3,3 mmol) TMEDA in 4 ml THF werden bei -78° C mit 3 mmol sec-BuLi versetzt. Nach 30 min gibt man 284 mg (1 mmol) der Verbindung aus 3-Cyclohexyl-1,2-ethandiol (gelöst in 1,5 ml THF) innerhalb von 25 min hinzu. Man versetzt die Reaktionsmischung nach 3 h in der Kälte mit 7 ml 2N Salzsäure und 10 ml Ether. Die Phasen werden getrennt, die wäßrige Phase zweimal ausgeethert und die vereinigten organischen Phasen mit gesättigter $NaHCO_3$-Lösung entsäuert. Nach dem Trocknen über Magnesiumsulfat wird das Lösemittel im Vakuum abgezogen und man erhält 524 mg eines Rohprodukts, dessen chromatographische Reinigung an Kieselgel mit Essigester/Hexan (1:2) 116 mg (26% der Theorie) des Diastereomeren Beispiel 18 und 126 mg (29% der Theorie) des Diastereomeres Beispiel 5 als farblose Öle ergibt.

Beispiel IV

$R_f$ = 0,55 (Essigester/Hexan = 1:1, Kieselgel)
$[\alpha]^{20}365$ = -10,1° (c = 1,1, Dichlormethan)
IR (Film): 3420 (OH), 3330 (NH), 1690 (OCON) und 1675 $cm^{-1}$ (OCON)

| $C_{25}H_{33}N_2O_5$ (441,55) | | |
|---|---|---|
| Ber.: | C 68,01 | H 7,53 |
| Gef.: | C 68,01 | H 7,76 |

Beispiel V

$R_f$ = 0,52 (Essigester/Hexan = 1:1, Kieselgel)
$[\alpha]^{20}D$ = -23,1° (c = 1,3, Dichlormethan)
IR (Film): 3420 (OH), 3330 (NH), 1690 (OCON) und 1675 $cm^{-1}$ (OCON)

| $C_{25}H_{33}N_2O_5$ (441,55) | | |
|---|---|---|
| Ber.: | C 68,01 | H 7,53 |
| Gef.: | C 68,09 | H 7,73 |

Beispiel VI

Cyclohexyl-1,2-ethandiol

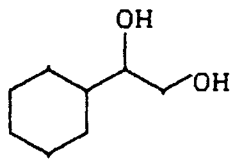

454 mg (2,0 mmol) der Verbindung aus (2-Hydroxy-2-cyclohexylethyl)-3,3-dimethyl-1-oxa-4-azaspiro-[4,5]decan-4-carboxylat wurden in 10 ml Methanol gelöst, 1 Tropfen Methansulfonsäure zugegeben und die Reaktionsmischung 15 h unter Rückfluß erhitzt. Nach dem Abkülhen wird der Ansatz mit einem starken Überschuß an Bariumhydroxid versetzt und weitere 4 h unter Rückfluß gekocht. Zur Aufarbeitung gießt man das abgekühlte Reaktionsgemisch auf je 10 ml 2N Salzsäure und Essigester, trennt die Phasen, extrahiert die wäßrige Phase zweimal mit Essigester, neutralisiert die vereinigten organischen Phasen mit gesättigter Natriumhydrogencarbonatlösung und trocknet über Magnesiumsulfat. Nach Flüssigkeitschromatographie an Kieselgel mit Essigester/Hexan = 1:1 erhält man 260 mg (1,8 mmol, 90% der Theorie) der Titelverbindung als farblosen Feststoff.
Schmp.: 40 ° C (Ether/Pentan)
$R_f$ = 0,22 (Essigester/n-Hexan = 1:1)
IR (KBr): 3330 $cm^{-1}$ (OH)

**Patentansprüche**

**1.** Verfahren zur Herstellung von Verbindungen der allgemeinen Formel (I)

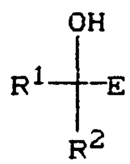

(I)

in welcher

$R^1$
- für Wasserstoff oder für eine elektrofuge Abgangsgruppe steht, oder
- für geradkettiges oder verzweigtes Alkyl oder Alkenyl mit jeweils bis zu 10 Kohlenstoffatomen steht, die gegebenenfalls durch Alkoxy mit bis zu 6 Kohlenstoffatomen , Phenyl oder durch die Gruppe $-NR^3R^4$ substituiert sind,
  worin
  $R^3$ und $R^4$ gleich oder verschieden sind und
  - Wasserstoff, geradkettiges oder verzweigtes Alkyl mit bis zu 8 Kohlenstoffatomen, Phenyl oder Benzyl bedeuten,

$R^2$
- für Wasserstoff steht, oder

$R^1$ und $R^2$ gemeinsam einen 3- bis 7-gliedrigen Cycloalkylring bilden

und

E
- für geradkettiges oder verzweigtes Alkyl oder Alkenyl mit jeweils bis zu 8 Kohlenstoffatomen steht, die gegebenenfalls bis zu 3-fach durch Hydroxy, Phenyl oder Cycloalkyl mit 3 bis 7 Kohlenstoffatomen substituiert sind oder durch eine Gruppe der Formel $-NR^3R^4$, $-HN-CO-OR^5$, $-SiR^6R^7R^8$ oder $SnR^{6'}R^{7'}R^{8'}$ substituiert sind,
  worin
  $R^3$ und $R^4$ die oben angegebene Bedeutung haben
  und
  $R^5$
  - geradkettiges oder verzweigtes Alkyl mit bis zu 6 Kohlenstoffatomen bedeutet, das gegebenenfalls durch Phenyl substituiert ist,

  $R^6$, $R^7$, $R^8$, $R^{6'}$, $R^{7'}$ und $R^{8'}$ gleich oder verschieden sind und geradkettiges oder

verzweigtes Alkyl mit bis zu 6 Kohlenstoffatomen bedeuten,

- für Cycloalkyl oder Cycloalkenyl mit 3 bis 7 Kohlenstoffatomen steht, das gegebenenfalls durch Hydroxy substituiert ist,
- für Carboxy, Alkoxycarbonyl mit bis zu 4 Kohlenstoffatomen oder für eine Gruppe der Formel $-SiR^6R^7R^8$, $-SnR^{6'}R^{7'}R^{8'}$ oder $R^9$-CO steht,

  worin

  $R^6$, $R^{6'}$, $R^7$, $R^{7'}$, $R^8$ und $R^{8'}$ die oben angegebene Bedeutung haben

  und

  $R^9$

  - Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel $-NH-CO-OR^5$ substituiert sind,

    worin

    $R^5$ die oben angegebene Bedeutung hat,

das dadurch gekennzeichnet ist, daß man Carbamate der allgemeinen Formel (II),

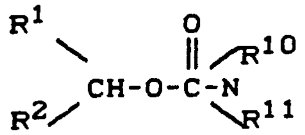

(II)

in welcher

$R^1$ und $R^2$ die oben angegebene Bedeutung haben

und

$R^{10}$ und $R^{11}$ gleich oder verschieden sind und entweder für geradkettiges oder verzweigtes Alkyl mit bis zu 8 Kohlenstoffatomen stehen, oder

gemeinsam mit dem Stickstoffatom für einen Rest der Formel

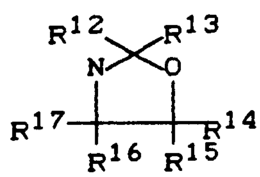

steht,

worin

$R^{12}$, $R^{13}$, $R^{14}$, $R^{15}$, $R^{16}$ und $R^{17}$ gleich oder verschieden sind und für Wasserstoff, geradkettiges oder ver-zweigtes Alkyl mit bis zu 8 Kohlenstoffatomen, Aryl mit 6 bis 10 Kohlenstoffatomen oder Cycloalkyl mit 3 bis 8 Kohlenstoffatomen stehen, oder jeweils $R^{12}$ und $R^{13}$, $R^{14}$ und $R^{15}$ und/oder $R^{16}$ und $R^{17}$ gemeinsam einen 3 bis 6 gliedrigen Carbocyclus bilden, in inerten Lösemitteln mit selektiven Basen, in Anwesenheit eines chelatbildenden, gegebenenfalls chiralen Diamins (im folgenden mit X bezeichnet), zu Verbindungen der allgemeinen Formel (III)

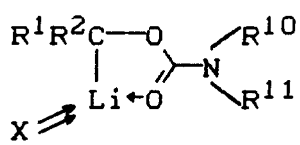

(III)

in welcher

$R^1$, $R^2$, $R^{10}$ und $R^{11}$ die oben angegebene Bedeutung haben,

quantitativ deprotoniert, was im Fall eines chiralen Diamins wie (-

)-Spartein enantioselektiv erfolgt,
und dann in inerten Lösemitteln, entweder mit achiralen Elektrophilen der allgemeinen Formeln (IV) und (V) oder mit gegebenenfalls prochiralen Elektrophilen der Formel (VI)

G'-Y (IV), $CO_2$ (V), 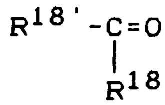 (VI),

in welchen

G
- die oben angegebene Bedeutung von E hat und mit diesergleich oder verschieden ist, aber nicht für Wasserstoff oder Carboxy steht,

Y
- für Halogen steht,

$R^{18}$ und $R^{18'}$ gleich oder verschieden sind und die oben angegebenen Bedeutungen von $R^9$ haben oder

$R^{18}$ und $R^{18'}$ gemeinsam einen Cyclobutyl-, Cyclopentyl- oder Cyclohexylring bilden

oder entweder $R^{18'}$ oder $R^{18'}$ für eine nukleofuge Abgangsgruppe steht,
substituiert,
und im Fall, daß $R^1$ für eine typische Abgangsgruppe steht, gegebenenfalls unter Ausbildung eines Acylanions und erneuter Deprotonierung nochmals elektrophil substituiert,
und in einem letzten Schritt, gegebenenfalls in Anwesenheit von Hilfsstoffen, die Schutzgruppe -CO-$NR^{10}R^{11}$ solvolytisch abspaltet.

**2.** Verfahren gemäß Anspruch 1 zur Herstellung von Verbindungen der allgemeinen Formel (I)

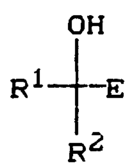 (I)

in welcher

$R^1$
- für Wasserstoff oder für die Gruppe

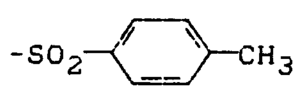

steht,
- für geradkettiges oder verzweigtes Alkyl oder Alkenyl mit jeweils bis zu 8 Kohlenstoffatomen steht, die gegebenenfalls durch Alkoxy mit bis zu 4 Kohlenstoffatomen, Phenyl oder durch die Gruppe $-NR^3R^4$ substituiert sind,
worin
$R^3$ und $R^4$ gleich oder verschieden sind und
  - Wasserstoff, geradkettiges oder verzweigtes Alkyl mit bis zu 6 Kohlenstoffatomen oder Benzyl bedeuten,

$R^2$
- für Wasserstoff steht, oder

$R^1$ und $R^2$ gemeinsam den Cyclopentylring bilden

und

E

- für geradkettiges oder verzweigtes Alkyl oder Alkenyl mit jeweils bis zu 6 Kohlenstoffatomen steht, die gegebenenfalls gleich oder verschieden bis zu 3-fach durch Hydroxy, Phenyl, Cyclobutyl, Cyclopentyl, Cyclohexyl oder durch eine Gruppe der Formel $-NR^3R^4$, -NH-CO-$OR^5$, $-SiR^6R^7R^8$ oder $-SnR^{6'}R^{7'}R^{8'}$ substituiert sind,
  worin
  $R^3$ und $R^4$ die oben angegebene Bedeutung haben,
  $R^5$
  - geradkettiges oder verzweigtes Alkyl mit bis zu 4 Kohlenstoffatomen bedeutet, das gegebenenfalls durch Phenyl substituiert ist,

  $R^6$, $R^{6'}$, $R^7$, $R^{7'}$, $R^8$ und $R^{8'}$ gleich oder verschieden sind und geradkettiges oder verzweigtes Alkyl mit bis zu 4 Kohlenstoffatomen bedeuten,
- für Cyclobutyl oder Cyclohexyl steht, die gegebenenfalls durch Hydroxy substituiert sind,
- für Carboxy Methoxycatbonyl oder für eine Gruppe der Formel $-SiR^6R^7R^8$, $-SnR^{6'}R^{7'}R^{8'}$ oder $R^9$-CO- steht,
  worin
  $R^6$, $R^{6'}$, $R^7$, $R^{7'}$, $R^8$ und $R^{8'}$ die oben angegebene Bedeutung haben
  und
  $R^9$
  - Wasserstoff oder geradkettiges oder verzweigtes Alkyl oder Alkenyl mit jeweils bis zu 6 Kohlenstoffatomen bedeutet, die gegebenenfalls bis zu 3-fach durch Phenyl oder durch die Gruppe der Formel -NH-CO-$OR^5$ substituiert sind,
    worin
    $R^5$ die oben angegebene Bedeutung hat.

**3.** Verfahren gemäß Anspruch 1, dadurch gekennzeichnet, daß man Verbindungen der allgemeinen Formel (I) herstellt,
in welcher
$R^1$
- für Wasserstoff oder Alkyl mit 1-4 C-Atomen steht,

$R^2$
- für Wasserstoff steht,

E
- für geradkettiges oder verzweigtes Alkyl oder Alkenyl mit jeweils bis zu 4 Kohlenstoffatomen steht, die gegebenenfalls gleich oder verschieden bis zu 3-fach durch Hydroxy, Phenyl, Cyclobutyl, Cyclohexyl oder durch eine Gruppe der Formel $-NR^3R^4$, -NHCO-$OR^5$, $-SiR^6R^7R^8$ oder $-SnR^{6'}R^{7'}R^{8'}$ substituiert sind
  worin
  $R^3$ und $R^4$ gleich oder verschieden sind und Wasserstoff, geradkettiges oder verzweigtes Alkyl mit bis zu 4 Kohlenstoffatomen oder Benzyl bedeuten,
  $R^5$
  - Methyl oder Ethyl bedeuten, die gegebenenfalls durch Phenyl substituiert sind,

  $R^6$, $R^{6'}$, $R^7$, $R^{7'}$, $R^8$ und $R^{8'}$ für Methyl stehen,
- für Cyclobutyl oder Cyclohexyl steht, die gegebenenfalls durch Hydroxy substituiert sind,
- für Carboxy, Methoxycarbonyl oder für eine Gruppe der Formel $-SiR^6R^7R^8$, $-SnR^{6'}R^{7'}R^{8'}$ oder $R^9$-CO- steht,
  worin
  $R^6$, $R^{6'}$, $R^7$, $R^{7'}$, $R^8$ und $R^{8'}$ die oben angegebene Bedeutung haben
  und
  $R^9$
  - Wasserstoff oder geradkettiges oder verzweigtes Alkyl oder Alkenyl mit jeweils bis zu 4 Kohlenstoffatomen bedeutet, die gegebenenfalls bis zu 3-fach durch Phenyl oder durch die Gruppe der Formel -NH-CO-$OR^5$ substituiert sind,
    worin
    $R^5$ die oben angegebene Bedeutung hat.

**4.** Verfahren gemäß Ansprüchen 1 bis 3, dadurch gekennzeichnet, daß man die Deprotonierung in einem Temperaturbereich von -100 °C bis Raumtemperatur durchführt.

5. Verfahren gemäß Ansprüchen 1 bis 4, dadurch gekennzeichnet, daß man als selektive Basen Alkyllithium-Verbindungen einsetzt.

6. Verfahren gemäß Ansprüchen 1 bis 3, dadurch gekennzeichnet, daß man die Abspaltung der Carbamatschutzgruppen bei Temperaturen zwischen 0°C und 130°C in Gegenwart von starken organischen oder anorganischen Säuren oder von Alkali- oder Erdalkalihydroxiden durchführt.